# EUROPEAN PATENT SPECIFICATION

(11) **EP 3 040 049 B1**
(45) Date of publication and mention of the grant of the patent: **26.08.2020**
(21) Application number: 14840885.9
(22) Date of filing: 29.08.2014
(51) Int. Cl.: A61C 19/045, A61B 5/11, G01B 3/04, A61B 5/107

(54) **OPENING MEASUREMENT INSTRUMENT**
ÖFFNUNGSMESSINSTRUMENT
INSTRUMENT DE MESURE D'OUVERTURE

(30) Priority: 30.08.2013 JP 2013180390
(43) Date of publication of application: 06.07.2016
(73) Proprietor: National University Corporation Tokyo Medical and Dental University, Bunkyo-ku Tokyo 113-8510 (JP)
(72) Inventor: YOSHITAKE, Hiroyuki, Tokyo 113-8510 (JP)
(74) Representative: Ström & Gulliksson AB
(86) International application number: PCT/JP2014/072764
(87) International publication number: WO 2015/030185

(56) References cited:
- GB-A- 2 494 232
- JP-A- 2004 329 431
- JP-U- 3 150 439
- JP-U- 3 150 439
- US-A- 4 997 368
- US-A- 4 997 368
- US-A- 5 035 616
- US-A- 5 158 096
- US-A- 5 361 506
- US-A- 5 361 506

## Description

### TECHNICAL FIELD

The present invention relates to a mouth-opening measuring instrument.

### BACKGROUND ART

Temporomandibular disorder is developed in a wide age range from children to adults. At present, the development of temporomandibular disorder is increasing rapidly particularly in young adults. One of main symptoms of temporomandibular disorder is lockjaw. Thus, a method of a remedy to temporomandibular disorder employs a mouth-opening training instrument disclosed for example in patent document 1. This mouth-opening training instrument is used in rehabilitation for recovering a mouth-opening function.

Test items adopted for a remedy to temporomandibular disorder include measurement of a mouth-opening amount. Specifically, the remedy using the mouth-opening training instrument and measurement of a mouth-opening amount are applied together. Meanwhile, measurement of a mouth-opening amount is an essential test item to be adopted not only in the remedy to temporomandibular disorder but also in general dental diagnosis and treatment. Thus, a mouth-opening amount is required to be measured simply and reliably.

A mouth-opening measuring instrument used conventionally for measuring a mouth-opening amount has a shape like a set square having scale marks given along each of two sides of the set square. A design for a dental ruler used for measuring the amount of mouth-opening between an upper row of teeth and a lower row of teeth is also known (patent document 2).
Patent Document 1: Japanese Patent No. 5004291
Patent Document 2: Design Registration No. 1369820

Further prior art documents disclosing mouth-opening measuring instruments include US 5361506 A, US 5035616 A, US 4997368 A and JP 3150439 U.

### DISCLOSURE OF THE INVENTION

### Problems to be Solved by the Invention

However, clear directions of use of the aforementioned mouth-opening measuring instrument are not given. For example, a criterion as to which part is to touch the instrument and which distance is to be measured is not established clearly. Regarding the aforementioned dental ruler, this ruler is to be used by inserting the ruler to the inner part of a dental arch width or applying the ruler to the outer part of the dental arch width and reading scale marks on the ruler. However, a criterion for measurement is also not established for this ruler. For these reasons, the aforementioned mouth-opening measuring instrument and the aforementioned dental ruler cannot be considered as being usable for fixed and stable measurement, so that they cannot be used stably during measurement. This reduces measurement accuracy and measurement reproducibility.

The present invention has been made in view of the aforementioned circumstances. It is an object of the present invention to provide a mouth-opening measuring instrument achieving excellent stability of the instrument during measurement, excellent measurement accuracy, and excellent measurement reproducibility.

### Means for Solving the Problems

Regarding measurement of a mouth-opening amount, the present inventor found that a mouth-opening amount can be measured properly by defining a tooth to be used as a reference for the measurement, providing a place where this tooth is to be fixed in a mouth-opening measuring instrument, and using this tooth as a fulcrum point during the measurement, thereby completing the present invention. More specifically, the present invention is defined in the appended independent claim.

The present invention provides a mouth-opening measuring instrument used for measuring the amount of mouth-opening between an upper jaw and a lower jaw, comprising: an engagement part to be engaged with a first reference tooth in one of the jaws; a measuring surface on which a second reference tooth in the other of the jaws is to abut while the engagement part is engaged with the first reference tooth; and first scale marks given on the measuring surface and indicating a distance from the engagement part.

The mouth-opening measuring instrument further comprises second scale marks given on the measuring surface and indicating a position in a direction perpendicular to a direction where the first scale marks are given.

The mouth-opening measuring instrument has a columnar shape with a curved part in cross section.

The mouth-opening measuring instrument may further comprise third scale marks indicating a forward glide amount of the first reference tooth relative to the second reference tooth.

The measuring surface includes a curved surface along which the measuring surface between the engagement part and a position on the measuring surface separated from the engagement part bulges out from a plane connecting the engagement part and the position on the measuring surface.

The curved surface may have a part increased in curvature with a greater distance from the engagement part.

The engagement part may comprise: a first reference tooth abutting surface on which the first reference tooth is to abut; and a recessed surface provided on at least one side of the first reference tooth abutting surface and recessed compared to the first reference tooth abutting surface.

The mouth-opening measuring instrument may further comprise gripping surfaces in a pair disposed in such a manner as to hold the measuring surface therebetween, wherein the griping surfaces in a pair include respective narrow surface parts separated by a width narrower than the width of the measuring surface in a direction where the second scale marks are given.

### Effects of the Invention

The present invention achieves excellent stability of the instrument during measurement, excellent measurement accuracy, and excellent measurement reproducibility.

### BRIEF DESCRIPTION OF THE DRAWINGS

FIG. 1 is a perspective view showing a mouth-opening measuring instrument according to a first embodiment of the present invention.
FIG. 2 is a side view showing the mouth-opening measuring instrument according to the first embodiment of the present invention.
FIG. 3 is a front view showing the mouth-opening measuring instrument according to the first embodiment of the present invention.
FIG. 4 shows a state where an upper jaw and a lower jaw are separated.
FIG. 5 shows a state where a mouth-opening measuring instrument 10 of the first embodiment is used while an engagement part of the mouth-opening measuring instrument 10 engages with the first reference teeth of FIG. 4 and the second reference teeth of FIG. 4 abuts on a measuring surface of the mouth-opening measuring instrument 10.
FIG. 6 shows a state where the mouth-opening measuring instrument 10 of the first embodiment is used while the engagement part of the mouth-opening measuring instrument 10 engages with the first reference teeth of FIG. 4 and the second reference teeth of FIG. 4 abuts on the measuring surface of the mouth-opening measuring instrument 10 while showing the absence of lateral displacement of a lower jaw.
FIG. 7 shows a state where the mouth-opening measuring instrument 10 of the first embodiment is used while the engagement part of the mouth-opening measuring instrument 10 engages with the first reference teeth of FIG. 4 and the second reference teeth of FIG. 4 abuts on the measuring surface of the mouth-opening measuring instrument 10 while showing the presence of lateral displacement of a lower jaw.
FIG. 8 is a side view showing a mouth-opening measuring instrument 10A according to a second embodiment of the present invention.
FIG. 9 shows a state where the mouth-opening measuring instrument 10A of the second embodiment is used while showing a state before first reference teeth glide forward relative to second reference teeth and a state after the first reference teeth glide forward relative to the second reference teeth.

### PREFERRED MODE FOR CARRYING OUT THE INVENTION

Preferred embodiments of a mouth-opening measuring instrument of the present invention are described next by referring to the drawings.

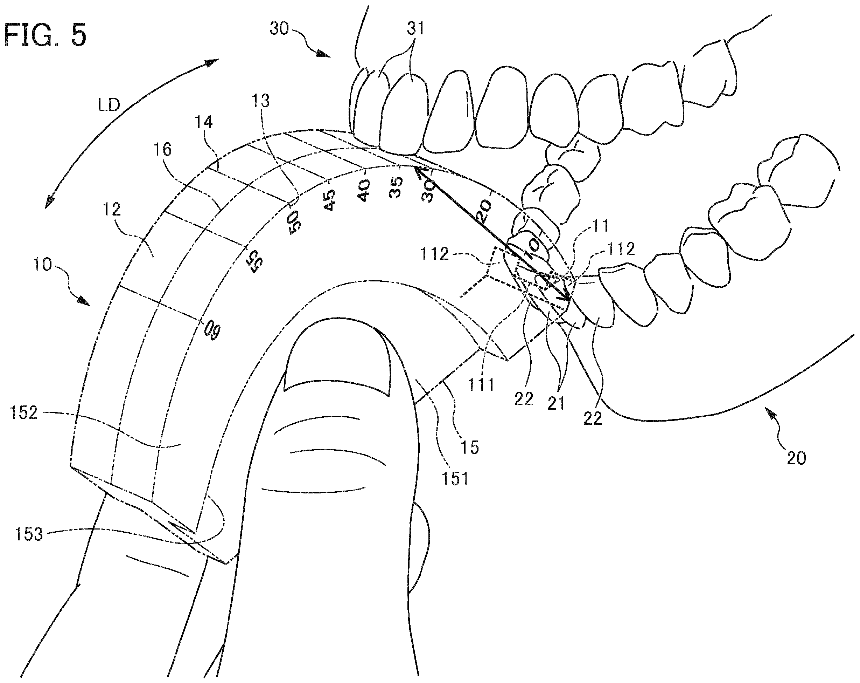

A mouth-opening measuring instrument 10 of a first embodiment is used for measuring the amount of mouth-opening between an upper jaw and a lower jaw. More specifically, the mouth-opening measuring instrument 10 is used for measuring a distance between front teeth in the lower jaw (first reference teeth 21 described later) and front teeth in the upper jaw (second reference teeth 31 described later) as a mouth-opening amount (see FIG. 5).

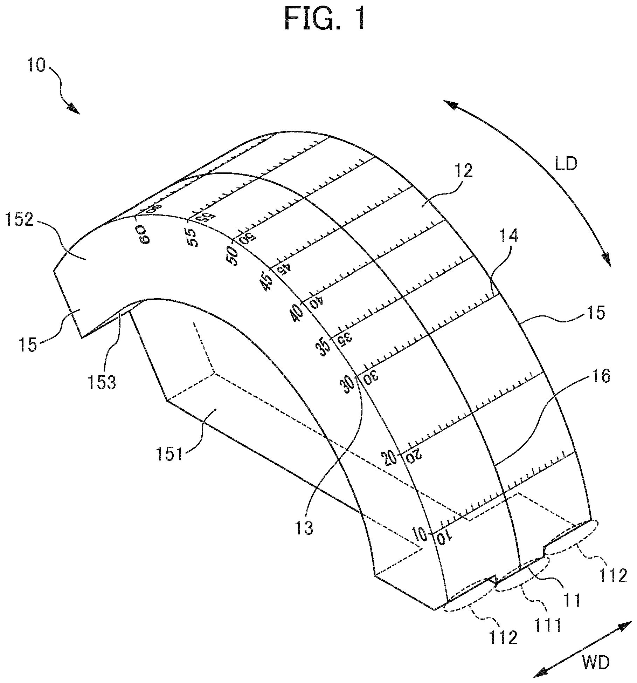

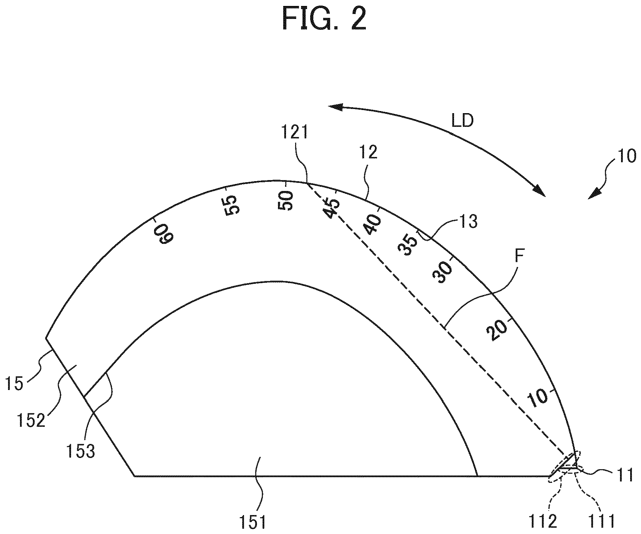

As shown in FIGS. 1 and 2, the mouth-opening measuring instrument 10 has a columnar shape in a form like a sector in cross section.

As shown in FIGS. 1 to 3, the mouth-opening measuring instrument 10 of the first embodiment includes an engagement part 11, a measuring surface 12, first scale marks 13, second scale marks 14, a reference line 16, and gripping surfaces 15 in a pair.

The engagement part 11 is provided at one end portion of a curved part of the mouth-opening measuring instrument 10 in a peripheral direction LD. As shown in FIGS. 1 to 3, the engagement part 11 includes a first reference tooth abutting surface 111 and recessed surfaces 112.

The first reference tooth abutting surface 111 is disposed in the center of the engagement part 11 in a width direction WD.

As shown in FIGS. 1 to 3, the recessed surfaces 112 are disposed on opposite sides of the first reference tooth abutting surface 111 in the width direction WD. The recessed surfaces 112 are recessed compared to the first reference tooth abutting surface 111.

As shown in FIGS. 1 and 2, the measuring surface 12 is provided in the curved part of the mouth-opening measuring instrument 10. As shown in FIG. 2, the measuring surface 12 includes a curved surface along which the measuring surface 12 between the engagement part 11 and a predetermined place 121 on a measuring surface (a position on the measuring surface 12 separated from the engagement part 11) bulges out from a plane F connecting the engagement part 11 and the predetermined place 121 on a measuring surface. While FIG. 2 shows only one predetermined place 121 on a measuring surface, the predetermined place 121 referred to in the first embodiment includes every place on the measuring surface 12 on which the second reference teeth 31 described later are to abut during measurement. More specifically, the measuring surface 12 includes a curved surface increased in curvature with a greater distance from the engagement part 11.

As shown in FIGS. 1 and 2, the first scale marks 13 are given on the measuring surface 12. The first scale marks 13 start from the engagement part 11, are separated at predetermined intervals along the measuring surface 12 in the peripheral direction LD, and extend in the width direction WD. The first scale marks 13 indicate a distance from the engagement part 11. The distance from the engagement part 11 mentioned herein means a linear distance from the engagement part 11 (first reference tooth abutting surface 111) to a point where the first scale mark 13 is given. This distance is a distance on the plane F shown in FIG. 2. In the first embodiment, the first scale marks 13 are given at intervals of 10 mm in a range where a distance from the engagement part 11 does not exceed 30 mm and at intervals of 5 mm in a range where this distance exceeds 30 mm.

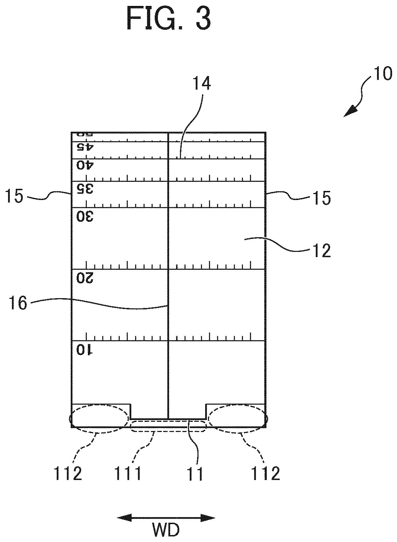

As shown in FIGS. 1 and 3, the second scale marks 14 are given at intervals of 1 mm in the width direction WD perpendicular to the peripheral direction LD of the measuring surface 12. The second scale marks 14 indicate a position in a direction perpendicular to a direction where the first scale marks 13 are given. In the first embodiment, the second scale marks 14 are given for a corresponding one of the first scale marks 13. The reference line 16 is disposed in the center of the measuring surface 12 in the width direction WD and extends in the peripheral direction LD of the measuring surface 12.

The reference line 16 functions as a reference position when a mouth-opening amount is measured using the mouth-opening measuring instrument 10.

As shown in FIGS. 1 to 3, the gripping surfaces 15 in a pair are disposed in such a manner as to hold the measuring surface 12 therebetween. As shown in FIG. 2, the gripping surfaces 15 each have a form like a sector in the width direction WD of the measuring surface 12. As shown in FIGS. 1 and 2, in the first embodiment, the gripping surfaces 15 each include a wide surface part 152, a narrow surface part 151, and a connecting surface part 153.

The wide surface part 152 is provided to extend continuously with the measuring surface 12 and is disposed perpendicular to the measuring surface 12. Specifically, a distance (width) between the wide surface parts 152 in a pair is equal to the length of the measuring surface 12 in the width direction WD.

The narrow surface part 151 is disposed adjacent to the central angle of the sector form of the gripping surface 15. The narrow surface parts 151 in a pair are disposed at a distance (separated by a width) shorter than the distance (width) between the wide surface parts 152 in a pair.

The connecting surface part 153 connects the wide surface part 152 and the narrow surface part 151. In the first embodiment, the connecting surface part 153 extends perpendicular to the wide surface part 152 and the narrow surface part 151.

A method of using the mouth-opening measuring instrument 10 according to the first embodiment is descried next.

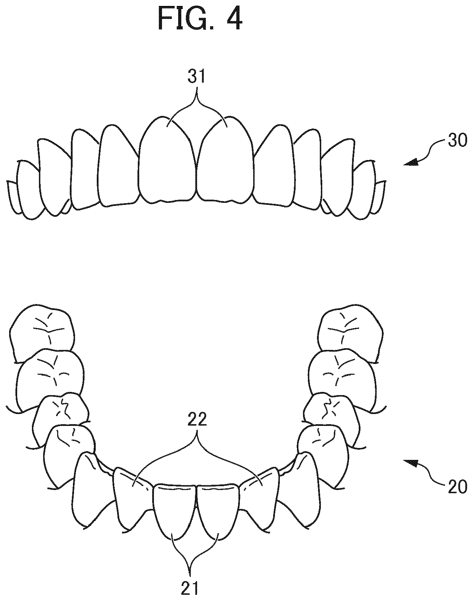

First, to measure the amount of mouth-opening between an upper jaw 30 and a lower jaw 20 using the mouth-opening measuring instrument 10 of the first embodiment, a test subject is made to open his or her mouth as shown in FIG. 4. Then, as shown in FIG. 5, the engagement part 11 of the mouth-opening measuring instrument 10 (more specifically, the first reference tooth abutting surface 111 formed by cutting as shown in FIGS. 2 and 3)is engaged with the first reference teeth 21 in the lower jaw 20. While the engagement part 11 is engaged with the first reference teeth 21, the measuring surface 12 is brought into abutting contact with the second reference teeth 31 in the upper jaw 30.

At this time, as the second reference teeth 31 of the test subject approach the measuring surface 12, mandibular incisors 22 positioned on opposite sides of the first reference teeth 21 are caused to abut on opposite sides of the first reference tooth abutting surface 111 easily. If the mandibular incisors 22 abut on opposite sides of the first reference tooth abutting surface 111, the first reference teeth 21 are obliged to move away from the first reference tooth abutting surface 111. This makes it impossible to engage the first reference teeth 21 stably with the first reference tooth abutting surface 111.

In this regard, in the first embodiment, the recessed surfaces 112 positioned on opposite sides of the first reference tooth abutting surface 111 are recessed compared to the first reference tooth abutting surface 111. This makes it unlikely that the mandibular incisors 22 will abut on the recessed surfaces 112 on opposite sides of the first reference tooth abutting surface 111 when the second reference teeth 31 approach the measuring surface 12. In this way, the first reference teeth 21 can be engaged stably with the first reference tooth abutting surface 111. As shown in FIGS. 4 and 5, the first reference teeth 21 are mandibular central incisors in the lower jaw 20. As shown in FIGS. 4 and 5, the second reference teeth 31 are maxillary central incisors in the upper jaw 30.

Next, as shown in FIG. 5, while the engagement part 11 is engaged with the first reference teeth 21 and the measuring surface 12 abuts on the second reference teeth 31, a distance from the engagement part 11 to a position on the measuring surface 12 on which the second reference teeth 31 abut is measured based on the first scale marks 13. This allows the second reference teeth 31 in the upper jaw to be supported stably on the measuring surface 12, so that the position of the second reference teeth 31 on the measuring surface 12 can be read accurately. As a result, a mouth-opening amount can be measured with a high degree of accuracy.

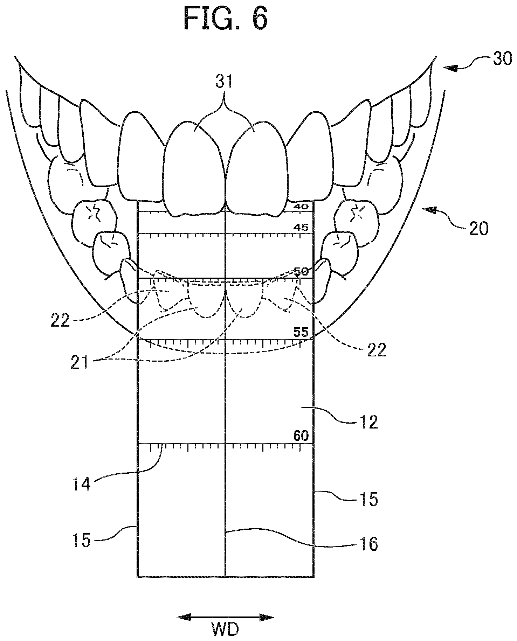

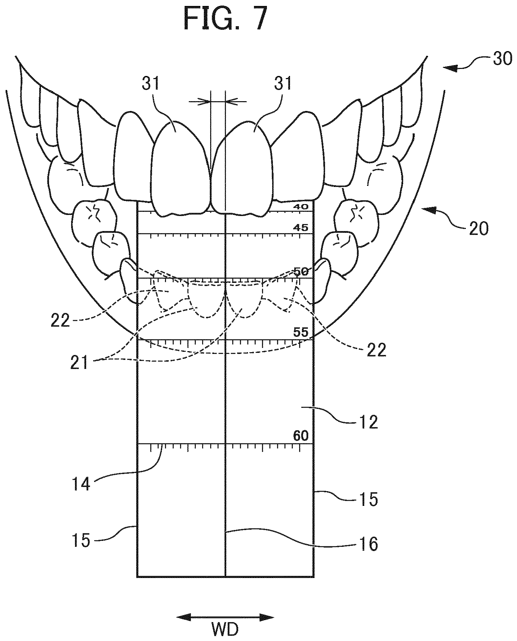

As shown in FIGS. 6 and 7, while the engagement part 11 is engaged with the first reference teeth 21 and the measuring surface 12 abuts on the second reference teeth 31, a distance from the reference line 16 to a place on the measuring surface 12 on which the second reference teeth 31 abut is measured based on the second scale marks 14. At this time, as shown in FIG. 7, a distance to a point between the two second reference teeth 31 of the abutting place of the second reference teeth 31 is measured. In this way, lateral displacement of mandibular incisors occurring while the mouth is opened can be measured as a discrepancy between the midline of maxillary incisors and the midline of the mandibular incisors. FIG. 6 shows a result of this measurement to the effect that lateral displacement of the mandibular incisors is not observed while the mouth is opened. FIG. 7 shows a result of this measurement to the effect that lateral displacement of the mandibular incisors is observed while the mouth is opened.

While the engagement part 11 is kept engaged with the first reference teeth 21 and the measuring surface 12 keeps abutting on the second reference teeth 31, the test subject is made to open his or her mouth gradually to continuously measure a place on the measuring surface 12 on which the second reference teeth 31 is abut based on the second scale marks 14. This makes it possible to record lateral displacement of the mandibular incisors continuously in response to a mouth-opening amount while the mouth is being opened, so that motions being made while the mouth is being opened can be understood.

The aforementioned mouth-opening measuring instrument 10 of the first embodiment achieves the following effects.

The mouth-opening measuring instrument 10 includes the engagement part 11 to be engaged with the first reference teeth 21 in a lower jaw, the measuring surface 12 on which the second reference teeth 31 in an upper jaw are to abut while the engagement part 11 is engaged with the first reference teeth 21, and the first scale marks 13 given on the measuring surface 12 and indicating a distance from the engagement part 11. This makes it possible to support the second reference teeth 31 in the upper jaw stably on the measuring surface 12, so that the position of the second reference teeth 31 on the measuring surface 12 can be read accurately. As a result, a mouth-opening amount can be measured with a high degree of accuracy. This further achieves excellent stability during measurement and measurement accuracy, thereby achieving a high degree of reproducibility.

If a discrepancy in mobility occurs between the right and left temporomandibular joints (for the reason that the forward gliding motion of one temporomandibular joint is disturbed, for example), a mouth-opening path displaces laterally. A mouth-opening measuring instrument conventionally used is dedicated to the function of measuring a distance between a maxillary incisor and a mandibular incisor and does not have the function of measuring the lateral displacement of the mouth-opening path properly in response to a mouth-opening amount to be caused by a discrepancy in mobility between the right and left temporomandibular joints. In the first embodiment, the mouth-opening measuring instrument 10 includes the second scale marks 14 given on the measuring surface 12 and indicating a position in a direction perpendicular to a direction where the first scale marks 13 are given. This makes it possible to measure lateral displacement of mandibular incisors occurring while a mouth is opened as a discrepancy between the midline of maxillary incisors and the midline of the mandibular incisors. This further makes it possible to record lateral displacement of the mandibular incisors continuously in response to a mouth-opening amount while the mouth is being opened, so that motions being made while the mouth is being opened can be understood.

If the measuring surface 12 of the mouth-opening measuring instrument 10 is a plane, the lips of a test subject are obliged to touch the mouth-opening measuring instrument 10 to make it difficult to make the measurement. In this regard, in the first embodiment, the measuring surface 12 of the mouth-opening measuring instrument 10 includes a curved surface along which the measuring surface between the engagement part 11 and the predetermined place 121 on a measuring surface bulges out from the plane F connecting the engagement part 11 and the predetermined place 121 on a measuring surface. This avoids touching of the lips of the test subject with the mouth-opening measuring instrument 10 during measurement, so that the measurement can be made easily.

If the measuring surface 12 of the mouth-opening measuring instrument 10 is flat, during measurement of lateral displacement of mandibular incisors while a mouth is opened, the measuring surface 12 is obliged to point in a direction opposite a measuring person as a mouth-opening amount increases. This makes it hard for the measuring person to recognize the second scale marks 14 visually. In this regard, in the first embodiment, the curved surface of the measuring surface 12 is formed to be increased in curvature with a greater distance from the engagement part 11. Thus, during measurement of lateral displacement of the mandibular incisors while the mouth is opened, a position on the measuring surface 12 on which the second reference teeth are abut is unlikely to point in the direction opposite the measuring person even if a mouth-opening amount increases. This allows the measuring person to visually recognize the second scale marks 14 easily.

The engagement part 11 includes the first reference tooth abutting surface 111 on which the first reference teeth 21 are to abut and the recessed surfaces 112 provided on opposite sides of the first reference tooth abutting surface 111 and recessed compared to the first reference tooth abutting surface 111. This can make it unlikely that the mandibular incisors 22 positioned on opposite sides of the first reference teeth 21 will contact the recessed surfaces 112 on opposite sides of the first reference tooth abutting surface 111 when the first reference teeth 21 are brought into abutting contact with the first reference tooth abutting surface 111. In this way, the first reference teeth 21 can be aligned correctly with the engagement part 11, so that a mouth-opening amount can be measured more accurately.

The mouth-opening measuring instrument 10 includes the gripping surfaces 15 in a pair disposed in such a manner as to hold the measuring surface 12 therebetween. The gripping surfaces 15 in a pair include the respective narrow surface parts 151 separated by a width narrower than the width of the measuring surface 12 in a direction where the second scale marks 14 are given. This allows a measuring person gripping the narrow surface parts 151 with his or her hand to hold the mouth-opening measuring instrument 10 easily, so that the measuring person can make measurement easily.

A mouth-opening measuring instrument 10A of a second embodiment differing from that of the first embodiment is described below. Points common to the first and second embodiments will not be described.

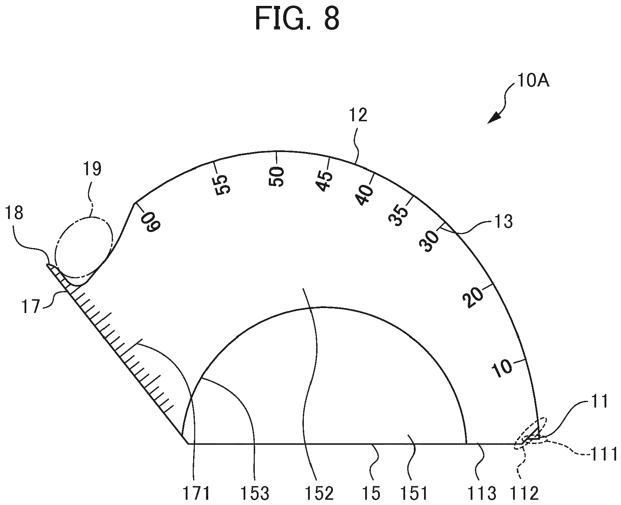

As shown in FIG. 8, the mouth-opening measuring instrument 10A includes a first horizontal surface 17, third scale marks 171, a flange part 18, and a cutout part 19 in addition to the aforementioned structures of the first embodiment.

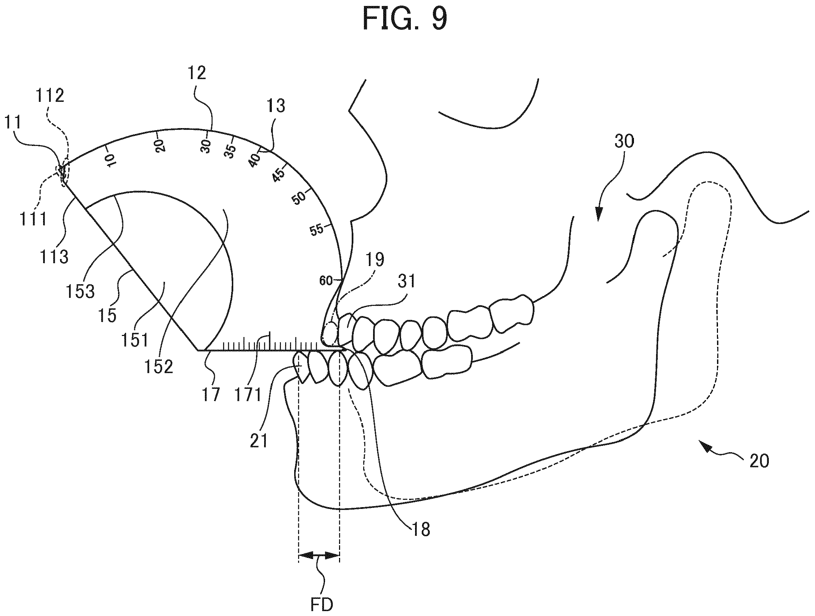

As shown in FIG. 9, the first horizontal surface 17 is provided on a surface on which the first reference teeth 21 are to abut during measurement of a forward glide amount FD described later. The first horizontal surface 17 is formed of a horizontal surface.

The third scale marks 171 are given on the first horizontal surface 17. The third scale marks 171 indicate the forward glide amount FD. More specifically, in the second embodiment, the third scale marks 171 indicate a distance from the tip of the flange part 18, as shown in FIG. 8. The forward glide amount FD shows a distance of the first reference teeth 21 from the second reference teeth 31. The forward glide amount FD corresponds to a distance of forward glide resulting from forward gliding motion of the lower jaw 20 of moving back and forth relative to the upper jaw 30. In the mouth-opening measuring instrument 10A of the second embodiment, the third scale marks 171 are given at intervals of 1 mm.

The forward glide amount FD is obtained as follows. As shown in FIG. 9, the second reference teeth 31 are placed on the flange part 18 described later. The first reference teeth 21 are brought into abutting contact with the first horizontal surface 17 in a state before the lower jaw 20 starts gliding. Then, the lower jaw 20 in abutting contact is caused to glide forward to measure a position on the first horizontal surface 17 of the first reference teeth 21 abut on after the glide. Next, a difference between the placement position of the second reference teeth 31 and the abutting position of the first reference teeth 21 is read, thereby obtaining the forward glide amount FD. In FIG. 9, the state of the lower jaw 20 before the glide is indicated by dotted lines of FIG. 9 and the state of the lower jaw 20 after the glide is indicated by solid lines of FIG. 9.

As shown in FIGS. 8 and 9, the flange part 18 of the mouth-opening measuring instrument 10A is provided in a part where the second reference teeth 31 are to be placed during measurement of the forward glide amount FD. The flange part 18 projects from the first horizontal surface 17 in a direction toward the part where the second reference teeth 31 are to be placed. In the mouth-opening measuring instrument 10A, the flange part 18 projects 5 mm. In this way, the flange part 18 is formed into a projecting shape in the mouth-opening measuring instrument 10A. This allows the second reference teeth 31 to be placed stably on the flange part 18 during measurement of the forward glide amount FD. As shown in FIG. 8, the third scale marks 171 reach the tip of the flange part 18. Meanwhile, in FIG. 9 the illustration of the third scale marks 171 is omitted from the flange part 18.

As shown in FIGS. 8 and 9, the cutout part 19 is disposed between the measuring surface 12 and the first horizontal surface 17. As shown in FIG. 9, the cutout part 19 has a shape recessed compared to the measuring surface 12 in order to prevent the upper lip of a test subject from touching the measuring surface 12 of the mouth-opening measuring instrument 10A and the second reference teeth 31 placed on the flange part 18 are housed in the cutout part 19 during measurement of the forward glide amount FD.

The aforementioned mouth-opening measuring instrument 10A of the second embodiment achieves the following effects.

The mouth-opening measuring instrument 10A includes the third scale marks 171 indicating the forward glide amount FD of the first reference teeth 21 relative to the second reference teeth 31. This makes it possible to measure the degree of forward gliding motion of the lower jaw 20 of moving back and forth relative to the upper jaw 30. Thus, the value of the forward glide amount FD and the value of the aforementioned mouth-opening amount can be used in combination for a test to be conducted for a remedy to temporomandibular disorder.

The mouth-opening measuring instrument 10A further includes the cutout part 19 having a shape recessed compared to the measuring surface 12. Thus, an upper lip is prevented from touching the mouth-opening measuring instrument 10A and the second reference teeth 31 placed on the flange part 18 are housed in the cutout part 19 during measurement of the forward glide amount FD. The flange part 18 is caught between the first reference teeth 21 and the second reference teeth 31, so that the mouth-opening measuring instrument 10A does not move up and down during measurement. This facilitates measurement, so that the measurement can be made with a high degree of accuracy. The forward glide amount FD can also be obtained by bringing the second reference teeth 31 into abutting contact with the tip of the flange part 18 and making the first reference teeth 21 glide forward. In this case, a position on the first horizontal surface 17 on which the first reference teeth 21 abut after the glide is measured and this measured position can be used as it is as the forward glide amount FD.

The present invention is not limited to the preferred embodiments of the mouth-opening measuring instrument according to the present invention but can be changed, where appropriate.

For example, in the first and second embodiments, for making measurements, mandibular central incisors are used as the first reference teeth and maxillary central incisors are used as the second reference teeth. However, this is not the only case. Specifically, the maxillary central incisors may be used as the first reference teeth and the mandibular central incisors may be used as the second reference teeth. In this case, with the engagement part engaged with the first reference teeth and the measuring surface abutting on the second reference teeth, a position on the measuring surface 12 on which the second reference teeth 31 is abut can be measured based on the first scale marks or the second scale marks. In this way, a mouth-opening amount and lateral displacement of the mandibular incisors can be measured while a mouth is opened.

For some test subjects, a lower jaw in an occlusal position may be displaced laterally. Even in this case, the mouth-opening measuring instrument of the present invention still allows measurement of the lateral displacement occurring while a mouth is opened, specifically, the amount of lateral displacement of a mouth-opening path for the lower jaw using the position of the lower jaw displaced laterally as a reference.

In the first and second embodiments, the measuring surface 12 is configured in such a manner that the predetermined place 121 on a measuring surface includes every place on the measuring surface 12 on which the second reference teeth 31 are to abut during measurement. However, this is not the only case. Specifically, the measuring surface 12 may also be configured in such a manner that the predetermined place 121 on a measuring surface includes one place on the measuring surface 12 on which the second reference teeth 31 are to abut during measurement.

In the first and second embodiments, the curved surface of the measuring surface 12 is formed to be increased in curvature with a greater distance from the engagement part 11. However, this is not the only case. Specifically, the curved surface of the measuring surface 12 may also be configured in such a manner that the curvature is increased in a part of the curved surface with a greater distance from the engagement part 11 while not being increased over the curved surface entirely with a greater distance from the engagement part 11.

In the first and second embodiments, the recessed surfaces 112 are positioned on opposite sides of the first reference tooth abutting surface 111 and recessed compared to the first reference tooth abutting surface 111. However, this is not the only case. Specifically, the recessed surface 112 may also be configured in such a manner as to be positioned on at least one side of the first reference tooth abutting surface 111 and recessed compared to the first reference tooth abutting surface 111.

A material used in the first and second embodiments is not particularly limited. For example, various synthetic resins or metal materials can be used.

In the first and second embodiments, the first scale marks 13 are given at intervals of 10 mm or 5 mm. However, this is not the only case. Specifically, the first scale marks 13 can be given at intervals such as 1 mm or 2 mm that are determined appropriately depending on purpose. For example, all the first scale marks 13 can be given at intervals of 1 mm and scale marks at intervals of 1 mm can also be given on the reference line 16. Then, numerical values in units of 5 mm can be assigned both to the scale marks 13 and the scale marks on the reference line 16.

The width of the second scale marks and the intervals of the second scale marks are also not limited but they can be determined appropriately depending on purpose.

In the second embodiment, the third scale marks 171 are given on the first horizontal surface 17. However, this is not the only case. What is required for measurement of the forward glide amount FD is only that the mouth-opening measuring instrument includes a surface on which the first reference teeth 21 can abut while the second reference teeth 31 abut on the mouth-opening measuring instrument, the surface on which the first reference teeth 21 can abut is a horizontal surface, and the lower jaw 20 can glide forward with the first reference teeth 21 abutting on this surface on which the first reference teeth 21 can abut. For example, the third scale marks 171 may be given on a second horizontal surface 113 of the wide surface part 152 disposed adjacent to the engagement part 11 and formed of a horizontal surface shown in FIG. 8. Alternatively, if a part of the measuring surface 12 is formed of a horizontal surface, the third scale marks 171 may be given on the horizontal part of the measuring surface 12. In either case, the forward glide amount FD can be measured by making the lower jaw 20 glide forward while bringing the second reference teeth 31 into abutting contact with a part of the second horizontal surface 113 adjacent to the engagement part 11 or with one end of the horizontal part of the measuring surface 12 and bringing the first reference teeth 21 into abutting contact with the horizontal part of the measuring surface 12 or the second horizontal surface 113. As described above, if the third scale marks 171 are given on the second horizontal surface 113 or the horizontal part of the measuring surface 12, the third scale marks 171 are provided in such a manner as to indicate a distance from a position on the second horizontal surface 113 or the horizontal part of the measuring surface 12 on which the second reference teeth 31 are to abut.

In the second embodiment, the third scale marks 171 are given at intervals of 1 mm. However, these intervals are not particularly limited but can be set appropriately depending on purpose.

In the second embodiment, the flange part 18 is configured to project 5 mm. However, the projection is not particularly limited. The flange part 18 may also be configured to project in a range from 1 to 10 mm, for example. The flange part 18 may also be configured not to project.

The flange part 18 may have additional scale marks similar to the second scale marks 14 given on a horizontal surface to touch the incisal edge of an incisor in the upper jaw 30. In doing this, deflection to the right and left (lateral displacement amount) occurring during forward glide can also be measured.

### EXPLANATION OF REFERENCE NUMERALS

- 10: Mouth-opening measuring instrument
- 10A: Mouth-opening measuring instrument
- 11: Engagement part
- 111: First reference tooth abutting surface
- 112: Recessed surface
- 113: Second horizontal surface
- 12: Measuring surface
- 121: Predetermined place on measuring surface
- 13: First scale mark
- 14: Second scale mark
- 15: Gripping surface
- 151: Narrow surface part
- 152: Wide surface part
- 153: Connecting surface part
- 16: Reference line
- 17: First horizontal surface
- 171: Third scale mark
- 18: Flange part
- 19: Cutout part
- 20: Lower jaw
- 21: First reference tooth
- 22: Mandibular incisor
- 30: Upper jaw
- 31: Second reference tooth
- LD: Peripheral direction
- WD: Width direction
- F: Plane
- FD: Forward glide amount

## Claims

1. A mouth-opening measuring instrument (10) used for measuring the amount of mouth-opening between an upper jaw and a lower jaw, comprising:
an engagement part (11) to be engaged with a first reference tooth in one of the jaws;
a measuring surface (12) on which a second reference tooth in the other of the jaws is to abut while the engagement part is engaged with the first reference tooth;
first scale marks (13) given on the measuring surface and indicating a distance from the engagement part; and
second scale marks (14) given on the measuring surface and indicating a position in a direction perpendicular to a direction where the first scale marks are given,
wherein the mouth-opening measuring instrument has a columnar shape with a curved part in cross section; and
wherein the measuring surface includes a curved surface along which the measuring surface between the engagement part and a position on the measuring surface separated from the engagement part bulges out from a plane (F) connecting the engagement part and the position on the measuring surface.

2. The mouth-opening measuring instrument according to claim 1, further comprising third scale marks indicating a forward glide amount of the first reference tooth relative to the second reference tooth.

3. The mouth-opening measuring instrument according to claim 1 or 2, wherein the curved surface has a part increased in curvature with a greater distance from the engagement part.

4. The mouth-opening measuring instrument according to any one of claims 1 to 3, wherein the engagement part comprises:
a first reference tooth abutting surface on which the first reference tooth is to abut; and
a recessed surface provided on at least one side of the first reference tooth abutting surface and recessed compared to the first reference tooth abutting surface.

5. The mouth-opening measuring instrument according to any one of claims 1 to 4, further comprising gripping surfaces in a pair disposed in such a manner as to hold the measuring surface therebetween,
wherein the griping surfaces in a pair include respective narrow surface parts separated by a width narrower than the width of the measuring surface in a direction where the second scale marks are given.

## Patentansprüche

1. Mundöffnungsmessgerät (10) zum Messen des Ausmaßes der Mundöffnung zwischen einem Oberkiefer und einem Unterkiefer, umfassend:
ein Eingriffsteil (11), das mit einem ersten Referenzzahn in einem der Kiefer in Eingriff gebracht wird;
eine Messfläche (12), an welcher ein zweiter Referenzzahn in dem anderen der Kiefer anliegen soll, während das Eingriffsteil mit dem ersten Referenzzahn in Eingriff steht;
erste auf der Messfläche angegebene Skalenmarkierungen (13), die einen Abstand vom Eingriffsteil angeben; und
zweite auf der Messfläche angegebene Skalenmarkierungen, die eine Position in einer Richtung senkrecht zu einer Richtung, in der die ersten Skalenmarkierungen angegeben sind, angeben,
wobei das Mundöffnungsmessgerät eine Säulen-artige Form mit einem im Querschnitt gekrümmten Teil aufweist, und
wobei die Messfläche eine gekrümmte Fläche aufweist, entlang der sich die Messfläche zwischen dem Eingriffsteil und einer vom Eingriffsteil getrennten Position auf der Messfläche von einer Ebene (F), die das Eingriffsteil und die Position auf der Messfläche verbindet, ausbaucht.

2. Mundöffnungsmessgerät nach Anspruch 1, ferner umfassend dritte Skalenmarkierungen, die ein Vorwärtsgleitausmaß des ersten Referenzzahns relativ zum zweiten Referenzzahn anzeigen.

3. Mundöffnungsmessgerät nach einem der Ansprüche 1 oder 2, wobei die gekrümmte Fläche einen Teil aufweist, dessen Krümmung mit einem größeren Abstand vom Eingriffsteil zunimmt.

4. Mundöffnungsmessgerät nach einem der Ansprüche 1 bis 3, wobei das Eingriffsteil umfasst:
eine erste Referenzzahn-Anlagefläche, an der der erste Referenzzahn anliegen soll; und
eine vertiefte Oberfläche, die auf mindestens einer Seite der ersten Referenzzahn-Anlagefläche vorgesehen und im Vergleich zur ersten Referenzzahn-Anlagefläche vertieft ist.

5. Mundöffnungsmessgerät nach einem der Ansprüche 1 bis 4, ferner umfassend Greifflächen in einem Paar, die so angeordnet sind, dass sie die Messfläche dazwischen halten, wobei die Greifflächen in einem Paar jeweils schmale Oberflächenteile umfassen, die durch eine Breite getrennt sind, die schmaler als die Breite der Messfläche in einer Richtung ist, in der die zweiten Skalenmarkierungen angegeben sind.

## Revendications

1. Instrument de mesure d'ouverture de bouche (10) utilisé pour mesurer la quantité d'ouverture de bouche entre une mâchoire supérieure et une mâchoire inférieure, comprenant :
une partie d'engagement (11) à engager avec une première dent de référence dans l'une des mâchoires ;
une surface de mesure (12) sur laquelle une seconde dent de référence dans l'autre des mâchoires doit buter tandis que la partie d'engagement est engagée avec la première dent de référence ;
des premières graduations (13) données sur la surface de mesure et indiquant une distance avec la partie d'engagement ; et
des deuxièmes graduations (14) données sur la surface de mesure et indiquant une position dans une direction perpendiculaire à une direction où les premières graduations sont données,
dans lequel l'instrument de mesure d'ouverture de bouche est de forme colonnaire avec une partie incurvée en section transversale ; et
dans lequel la surface de mesure comporte une surface incurvée le long de laquelle la surface de mesure entre la partie d'engagement et une position sur la surface de mesure séparée de la partie d'engagement déborde hors d'un plan (F) reliant la partie d'engagement et la position sur la surface de mesure.

2. Instrument de mesure d'ouverture de bouche selon la revendication 1, comprenant en outre des troisièmes graduations indiquant une quantité de glissement vers l'avant de la première dent de référence par rapport à la seconde dent de référence.

3. Instrument de mesure d'ouverture de bouche selon la revendication 1 ou 2, dans lequel la surface incurvée a une partie de courbure accrue avec une distance plus grande avec la partie d'engagement.

4. Instrument de mesure d'ouverture de bouche selon l'une quelconque des revendications 1 à 3, dans lequel la partie d'engagement comprend :
une surface de butée de première dent de référence sur laquelle la première dent de référence doit buter ; et
une surface en retrait prévue sur au moins un côté de la surface de butée de première dent de référence et en retrait en comparaison à la surface de butée de première dent de référence.

5. Instrument de mesure d'ouverture de bouche selon l'une quelconque des revendications 1 à 4, comprenant en outre des surfaces de préhension en paire disposées de manière à maintenir la surface de mesure entre elles,
dans lequel les surfaces de préhension en paire comportent des parties de surface étroites respectives séparées par une largeur plus étroite que la largeur de la surface de mesure dans une direction où les deuxièmes graduations sont données.
